# EUROPEAN PATENT SPECIFICATION

(11) **EP 0 683 642 B1**
(45) Date of publication and mention of the grant of the patent: **20.01.1999**
(21) Application number: 93917718.4
(22) Date of filing: 02.08.1993
(51) Int. Cl.: A61B 5/024, A61B 5/00

(54) **SENSOR AND TRANSMITTER FOR PHYSIOLOGIC SIGNALS**
MESSAUFNEHMER UND SENDER FÜR PHYSIOLOGISCHE SIGNALE
CAPTEUR ET EMETTEUR DE SIGNAUX PHYSIOLOGIQUES

(30) Priority: 31.07.1992 IT VA920018
(43) Date of publication of application: 29.11.1995
(73) Proprietor: MURRI, Vittorio, I-21053 Castellanza (IT)
(72) Inventor: MURRI, Vittorio, I-21053 Castellanza (IT)
(74) Representative: Sassi, Romano
(86) International application number: EP9302050
(87) International publication number: WO9403105

(56) References cited:
- DE-A- 2 815 395
- DE-A- 2 824 899
- DE-A- 2 845 679
- US-A- 3 572 316
- US-E- R E32 275

## Description

The present invention relates to devices for surveying (measuring, detecting, monitoring, estimating, inspecting etc.), particularly permanently, in real time, signals having diagnostic meaning and for revealing them at a close and/or remote distance, in any available perceptible and /or technical form. E.g. they may comprise a warning device to detect and notify in real time, at distance and/or in a permanently receivable form by the seeker, the present body temperature, of a potential patient, particularly unable to manifest himself about his status, such as children and babies or seriously ill persons, as well as adapted to provide, one or more warning signals e.g. in acoustic or radio-frequency form and/or manageable as stored data for diagnostic and/or therapeutic purposes, particularly when the temperature exceeds prefixed limits.

At the present state of the art a number of apparatus for detecting signals having diagnostic meaning, even having shape of bracelet, are known. US-A-3572316 disclose a device for survying , particularly permanently, in real time, signals having diagnostic meaning and for revealing them at a remote distance. The signals are revealed in a perceptible form in a technical available fashion. The known device comprises also a carrier in the form of wrist watch, at least one sensory equipment providing signals having diagnostic meaning at least an emitter and a power economizer. DE-A-2824899 disclose the alternative of revealing the signals directly at a close distance in combination with other features .

These devices are critical in that no means are provided to ensure a soft, even and, constant contact and pressure between the user's skin and the sensor. Due to this fact the monitoring was vitiated and the results untrustable.

The invention as claimed is intended to remedy these drawbacks. The inventor, in a device for surveying, particularly permanently, in real time, signals having diagnostic meaning and for revealing them at a close and/or remote distance, in any available perceptible and/or technical form, comprising a carrier, substantially in the form of wrist watch, at least a sensory equipment adapted to provide signals having diagnostic meaning, at least a directly or indirectly organolectic disclosing equipment, and a power economiser with ingenious perception, have conceived to place the proper sensor at the end of a flexible arm, in order that, by wearing the bracelet, the said sensor adheres to the subject's skin, with a pressure suitable to provide a correct detection.

The body temperature sensory equipment, is particularly an electronic temperature probe, of the demi-cylindrical kind and which is placed on the flexible arm in such a way that the user's skin contact area corresponds to the even surface, wherein the sensing cells of the same probe are placed. Moreover the thermoprobe is provided with a flexible bell-like enclosure in the form of a corolla of petals, whereby such petals may adhere to the skin, due to their own elasticity and to the pressure applied, substantially providing the thermoprobe with a constant temperature environment.

In accordance with a preferred embodiment of the present invention, the flexible arm cooperates with a switch excluding the battery, controlled by the swinging of the flexible arm, whereby the switch is off when the flexible arm is released.

Unlike from the detecting equipment, the disclosing equipment displaying a signal in any perceptible form, comprises at least a transducer of the detected signal and at least an emitter of the converted signal. It comprises more signal transducers, e.g., five, as many as the human senses. Like the detecting equipment, the disclosing equipment comprises a commutation switch, having a number of poles equal to the number of transducers, provided upstream of transducers. At least one of the transducers, is a radio-frequency transducer provided downstream with an antenna, particularly an I/O radio antenna. The transducer of the signal is placed on at least a demi-remote equipment and/or on at least an equipment remote from the bracelet-like component. More signal transducers and a commutation switch, having a number of poles equal to the number of transducers, provided upstream of transducers, may be installed on a demi-remote and/or remote unit. At least one of transducers is a radio-frequency transducer provided downstream with an I/O radio antenna. On a demi-remote and/or remote unit may be installed a power repeater equipment, whereby to limit the power consumption on the disclosing equipment. The demi-remote and/or remote unit is provided with means connectable to any network such as power, telephone, radio frequency, digital, optical-fibre networks and the like.

This signal may be immediately utilized or elaborated, before, during or after the broadcasting and/or stored for future use and/or converted into message of the kind receivable by the seeker. E.g. the seeker could be a non seeing person for deficiency of sight or of illumination, who is in the need to receive acoustically the sought data reading out. Moreover, in relation to the accuracy of the reading the errors are insignificant, the significant ones could arise from an imperfect contact of the temperature sensor with the body, of which the correct temperature is to be monitored.

In accordance with another preferred embodiment of the present invention, in the processing and use of the sensor signal and, may be foreseen the harmonization of the signals with the basal temperature of the patient, in order that such signals cease to have absolute meaning, but only relative, commensurate with the user according to his age, his particular condition and for the duration of this condition. Moreover, this device could be associated to a storing system to store data which are meaningful for the course of illness, in order that the transient temperatures do not represent a reason to worry but, on the contrary, in case that the value of the data is descending, a reason of relief and happiness Thus such data could be duly organized for the edition of a report or for plotting a diagram, regarding a time interval which can be selected for transmission, even to a remote distance. Of course, in consideration of the substantially permanent, automatic, operation of the instruments, according to the present invention, their application is substantially unlimited; as such not conditioned by casualty, i.e., by the presence of the symptom and/or its perception. Thus, just the permanent use of the device provides, not only the symptoms, but a chronological report of the course of the data having diagnostic meaning, accompanying or following the various happenings of the life, so as to represent essential or at least significative components of the biorythm. In this context the individual user may be able to do important diagnostic significative self-controls, such as checking woman ovulation. Moreover the various warning devices substantially available and commutable may be of decisive help even for people without one or more senses, commuting the disclosing equipment on the emitter inducing the effect perceivable by that particular person.

One way of carrying out the invention is described in detail below, with reference to drawings, which illustrate a specific embodiments, in which:
Figure 1 is a flowchart, of a warning device, according to the present invention, comprising the detecting means and the disclosing means connected to transreceiver means.
Figure 2 schematizes the outer materialization of the hardware of the flowchart of figure 1 and having substantially the outer shape of a wrist watch.

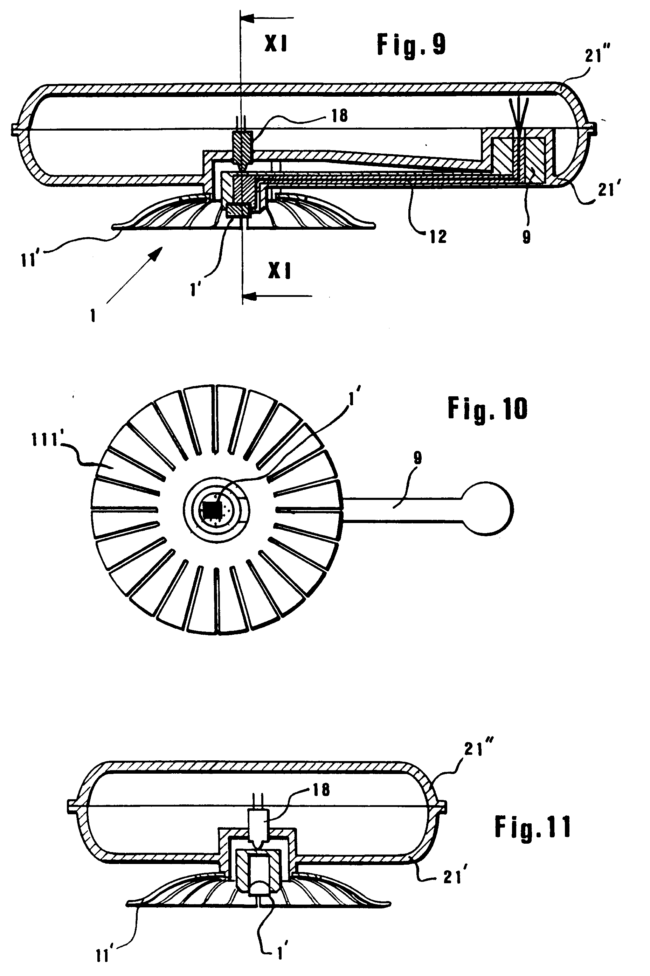

Figure 3 is a flowchart referring to a disclosing equipment of a warning device wherein the detecting means are separated from the disclosing means and the former are subrogated by transreceiver means. In figures 4 to 10 as well in this one the disclosing or receiving means are provided with chrono-thermo-memory as well as with a monitor, to display chrono-thermal intervals stored therein, in the form of curves, in a system of cartesian coordinates.
Figure 4 schematizes the outer materialization of the flowchart of figure 3.
Figure 5 is substantially a repetition of figure 3, but referring to a multiple disclosing equipment which is provided with a single display for reading out each time the data regarding one of the patients under observation which may be selected by switching on him.
Figure 6 schematizes the outer materialization of the flowchart of figure 5.
Figure 7 is substantially a repetition of figure 5, but referring to a multiple disclosing equipment provided with a contemporary display of the data of all the patients undergoing the monitoring or checking
Figure 8 schematizes the outer materialization of the flowchart of figure 7.
Figure 9 is a detailed cross section, in an enlarged scale, of the casing of the device of figure 2, wherein the cross-section is taken along the line IX-IX of figure 2 and wherein the sensor provided at the end of the flexible arm is shown.
Figure 10 is is a plane view of the bottom side of the flexible arm carrying the sensor provided at its end and means connected thereto of figure 9.
Figure 11 is a cross section, in an enlarged scale, taken at the end of the flexible arm carrying the sensor and the means connected thereto, wherein the cross-section is taken along the line XI-XI of figure 9.
Figure 12 is a flowchart of a demi-remote interface or power repeating equipment, provided as component, particularly permanent, to interface, with crossbar controls, essentially, the detecting means and the disclosing means and instrumentally the various power, signal and the like networks.

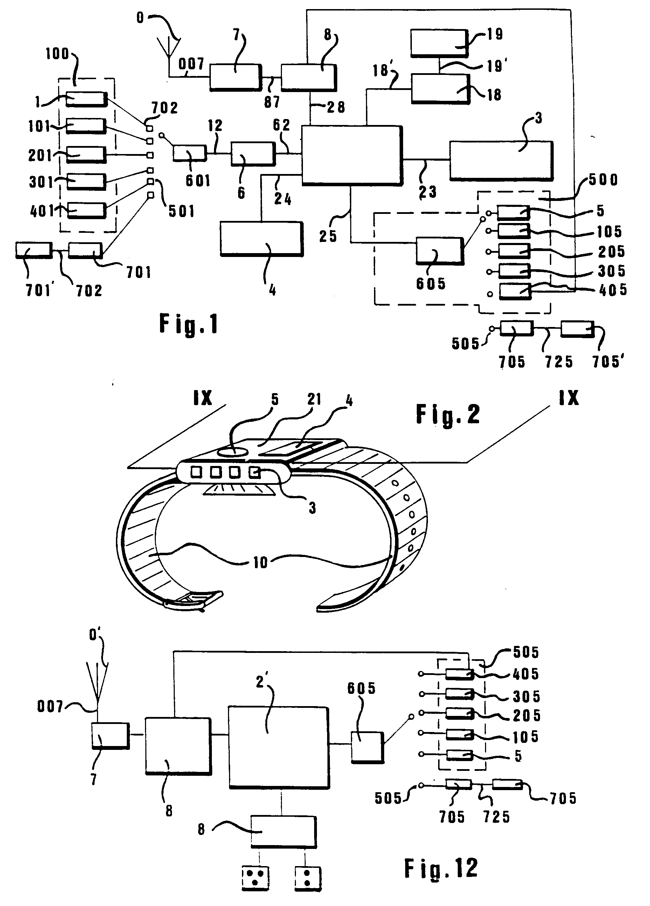

Referring now to figure 1 of the drawings, a warning device, according to the present invention, includes: a sensory equipment 100, a microprocessor 2, a keyboard 3, a display 4, a disclosing equipment 500, a battery 19, an economiser switch 18, a V-f converter 6, a transreceiver duplexer 7 and a modem 8, as well as an I/O radio antenna 0. The sensory equipment 100, of which it will be spoken later on, is connected, by connection 12, through the V-f converter, 6 and by connection 62 to the microprocessor 2, which through connection 28 is connected to the modem 8, which, in turn, is connected, through connection 87, to a transreceiver duplexer 7, which is duly provided with an antenna 0 and with a connection 007. The microprocessor 2, is connected: through connection 23, to the keyboard 3; through connection 24, to the display 4; and, through connection 25, to the disclosing equipment 500, of which it will be spoken later on. The components of the sensory equipment 100, convert the quantities detected into electric current signals. Each electric current signal attains the microprocessor 2: through the wire 12, as well as through the V-f converter 6 and the connection 62, in the form of frequency signal. The microprocessor 2 processes the value of this frequency and in function thereof provides instructions to be sent, through the wire 28, to the modem 8. Other instructions, are sent in the form of digital pulses, through the wire 87 to a transreceiver duplexer 7. The latter transmits modulated radio frequency pulses, in function of the digital pulses received, through the wire 007, to the antenna 0. The antenna 0, in turn, receives radio frequency signals which, following the opposed described course, reach the microprocessor 2, in form of digital pulses. In other words, the microprocessor 2, from one side processes the remote instructions received from the antenna and from the other side the signals of frequency relating to the quantity monitored by the local sensor and provides, through the transreceiver duplexer 7 and the antenna 0, the broadcasting of the appropriate answers to one or more other antennas 0', to be described later on.

The attribute organolectic, assigned to the detecting equipment 100 and to the disclosing equipment 500, means that the sensory equipment can monitor the signal in any perceptible form, thus, for a thorough monitoring, it is provided with five components 1, 101, 201, 301, 401, symbolically representing the five human senses. In practice these are: the body temperature, sensory equipment 1 (touch), cardiacal frequency sensory equipment 101 (hearing), pH of the sweat sensory equipment 201 (taste), muscular contractility sensory equipment 301 (sight), detecting of organic gases sensory equipment 401 (olfaction). Of course for the use, each time, of the necessary sensory equipment, a commutation switch 601 is provided, of which at least one pole (501) is comprised by a plug, for the connection of at least one pole (701) of a sensor (701') applied to a free pole of a cable (702). This permits to acquire, through a free probe, any significative signal going to appraise it anywhere it is available, E.g., a vaginal probe for the thermocontrol of the ovulation. In a similar way the disclosing equipment 500 may detect the signal in any appreciable form, thus for being complete is provided with five components 5, 105, 205, 305, 405, symbolically representing the five human senses. In practice, an emitter 5 of vibrations (touch), an acoustic emitter.105 (hearing), an emitter 205 of tasty substance (taste), emitter 305 of luminous signal (sight), an emitter 405 of odour (olfaction). Of course, for using each time the wanted emitter a commutation switch 605 is provided, at least one pole 505 of which is comprised by aplug, for the connection of at least one pole 705 of an emitter 705' provided at the free pole of a cable 725. This offers the possibility to acquire, any significative signal whereby to carry it where it may be perceived.

The microprocessor 2, then, processes the value of the current of the sensor and in function of it emits digital signals to be sent, through the wire 24, to the display 4, as well as through the wire 25 to the organolectic disclosing equipment 500, of which it will be spoken later on. Thus, the signal which attains the monitor 4 displays the temperature value detected by the sensory equipment, while the signal reaching the organolectic disclosing equipment 500, qualify the same to the emission of the signal which, e.g., may be the recitation of the temperature indicated by the display. The qualification of the acoustic emitter 5 is necessary but not sufficient condition for the emission of the signal. In fact the signal is emitted provided that an instruction regarding the significance of the acoustic signal was sent, through the keyboard 3, and in turn, through the wire 23', to the microprocessor 2. Of course, the significance wanted or ought may be typed on the keys of the keyboard 3, in order that the microprocessor can duly intervene.

Referring now to figure 2 and to the figures from 9 to 11 of the drawings, a warning device, having substantially the shape of a bracelet 10, having, as central section, a casing 21, which comprises: a sensory equipment or thermoprobe 1, to be described in details later on, a microprocessor 2, not shown, a keyboard 3, a display 4, and an acoustic emitter 5.

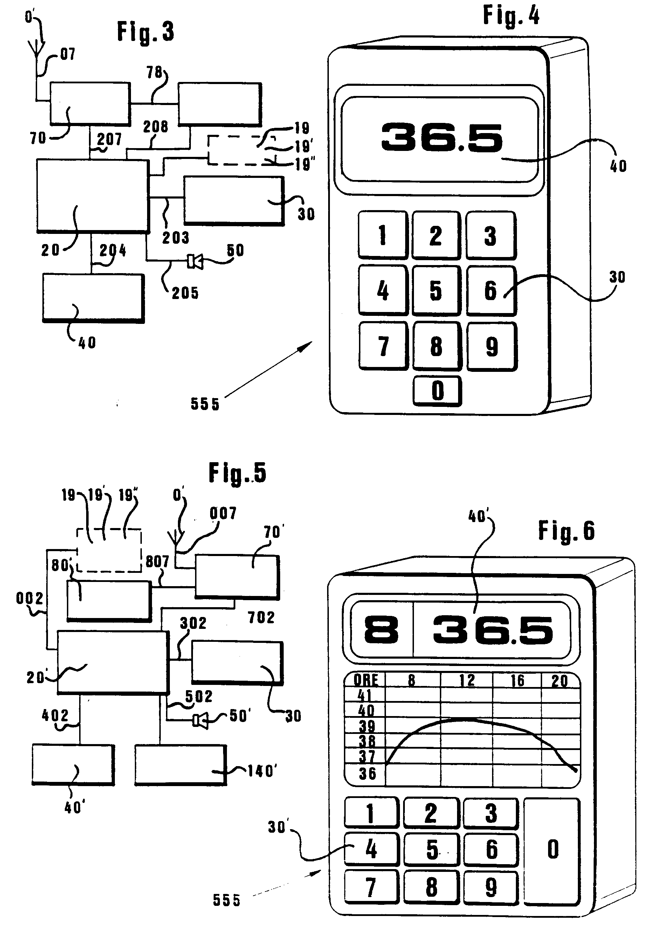

Referring now to figure 3 of the drawings, the disclosing equipment flowchart in accordance with the simplest embodiment of the present invention, comprises: a microprocessor 20, a keyboard 30, a display 40, a transreceiver duplexer 70, a modem 80 and an acoustic emitter 50, as well as an I/O radio antenna 0'. Such antenna 0' provides an input or output signal which, through the connection 007, interacts with the transreceiver duplexer 70, which, from one side, through connection 207 is connected to the microprocessor 20, while from the other side, through the connection 78 is connected to the modem 80. In turn, the microprocessor 20 is connected: through a connection 208, to the modem 80, through a connection 203 to the keyboard 30, through connection 205 to the acoustic emitter 50 and through connection 204 to display 40. The electromagnetic pulses impinging the antenna 0', particularly those coming from antenna 0, which provides a transreceiver dialogue with the same antenna 0', run, in both directions, along the wire 007 for impinging, in both conditions, the transreceiver duplexer 70, which, in both conditions, interacts with through the wire 78, the modem 80, which dialogues through the wire 208 with the microprocessor 20. The microprocessor 20 therefore dialogues with all what described and obviously even with the antenna 0 and after all with the microprocessor 2 for the reason to be explained later on. On the other hand microprocessor 20 is controlled by keyboard 30, through the wire 203 and controls, through the wire 205, the acoustic emitter 50 and through the wire 204 the display 40.

Referring now to figure 5 of the drawings, a disclosing equipment flowchart, in accordance with a preferred embodiment of the present invention, comprises: a microprocessor 20', a transreceiver duplexer 70', a modem 80', a keyboard 30', a digital display 40', a graphic display 140' and an acoustic emitter 50', as well as an I/O radio antenna 0'. Such antenna 0' provides an input or output signal which, through the connection 007, interacts with on a transreceiver duplexer 70', which, from one side, through connection 702, is connected to said microprocessor 20', while from the other side through the connection 807 is connected to the modem 80'. In turn the microprocessor 20' is connected: through connection 802, back, to the modem 80', through connection 302 to the keyboard 30', through connection 502 to the acoustic emitter 50', through connection 402 to display 40' and through connection 142 to the graphic display 140'. Even in this figure the electromagnetic pulses interacting with the antenna 0', particularly those broadcast by antenna 0, which provides a transreceiver dialogue with the same antenna 0'; run, in due both directions, along the wire 007 to involve, in both conditions, the transreceiver duplexer 70', which, in both conditions, interacts through the wire 807, with the modem 80', which dialogues, through the wire 802 with microprocessor 20'. The microprocessor 20' therefore dialogues with all what described and obviously, also with the antenna 0 and at the end with the microprocessor 2 for the reason to be explained later on. On the other hand the microprocessor 20' is controlled by the keyboard 30' through the wire 302 and controls, through the wire 502, the acoustic emitter 50', through the wire 402 the display 40' and through the wire 142, the display 140'.

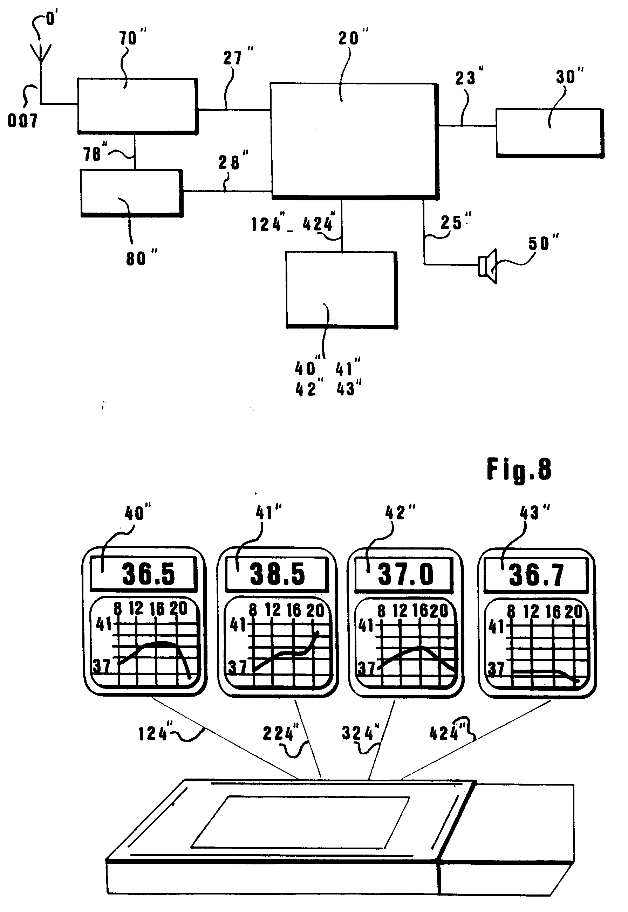

Referring now to figure 7 of the drawings, the disclosing equipment a flowchart, in accordance with one embodiment of the present invention, comprising a common detector, comprises: a microprocessor 20'', a transreceiver duplexer 70'', a modem 80'', a keyboard 30'', a series of displays 40'', 41'', 42'', 43'' and an acoustic emitter 50'', as well as an I/O radio antenna 0'. Such antenna 0' provides an input or output signal which, through the connection 007 interacts with the transreceiver duplexer 70'', which, from one side, through connection 27'' is connected to the microprocessor 20'', while from the other side, through the connection 78'' is connected to the modem 80''. In turn the microprocessor 20'' is connected: through the connection 28'', back, to the modem 80'', through the connection 23'' to the keyboard 30'', through the connection 25'' to the acoustic emitter 50'', through the connections 124'', 224'', 324'', 424'' to the displays 40'', 41'', 42'', 43''. Even in figure 7 the electromagnetic pulses interacting with the antenna 0', particularly those coming from the antenna 0 which provides a transreceiver dialogue with the same antenna 0' run, in both directions, along the wire 007 for interacting, in both conditions, with the transreceiver duplexer 70'', which, in both conditions, interacts, through the wire 78'', with the modem 80'', which dialogues, through the wire (27'') 28'' with the microprocessor 20''. The microprocessor 20'' therefore dialogues with all what described and obviously even with the antenna 0 and after all with the microprocessor 2' for the reason to be explained later on. On the other hand the microprocessor 20'' is controlled by the keyboard 30'', through the wire 23'' and controls, through the wire 25'' the acoustic emitter 50'' and through the four wires 124, 224, 324, 424, the four wires 40'', 41'', 42'' 43''.

Of course all over the figures from 1 to 10 are provided one or more members: power supply 19, switch 19', warning light 19'', possibly shown only in the even figures. Whereas they are shown together within the same dashed box in the odd figures.

From what described hereabove the operation of the warning device in all its forms should be evident, however hereafter is given a short description. Suppose that the bracelet- like device is provided with all the sensory equipments 1, 101, 201, 301, 401, and that it is provided with a probe. More particularly, suppose that the body temperature may be surveied by sensory equipment 1 (touch), the cardiacal frequency by sensory equipment 101 (hearing), the pH of the sweat by sensory equipment 201 (taste), the muscular contractility by sensory equipment 301 (sight), the detecting of organic gases by sensory equipment 401 (olfaction). Moreover that the probe serves to measure the blood pressure. Through the commutation switch 601, the detection wanted is selected. Of course the microprocessor controls the conformity of the signal and possibly stores it, making it available to the emission in the corresponding form. As in the detecting equipment 100 the signal obtained may be revealed in any appreciable form; thus for completion reasons the disclosing equipment is provided with five components 5, 105, 205, 305, 405, symbolically representing the five human senses. In practice, a vibration emitter 5 (touch), an acoustic emitter 105 (hearing), an emitter 205 of tasty substance (taste), an emitter 305 of luminous signal (sight), an emitter of smell 405 (olfaction). Of course, for utilizing each time the wanted emitter the disclosing equipment 5 is provided with a commutation switch 605, at least one pole 505 of which is comprised by a plug, for the connection with at least one pole 705 of an emitter 705', provided at the free pole of a cable 725. This offers the possibility of acquiring, any significative signal carrying it where it is perceivable.

If, from one side, the wrist-watch-like carrier shall have small dimensions and a lower power consumption in order that, with a battery of small dimensions a relatively long life is possible and from the other side, a maximum power of transmission is wanted, a demi-remote (figure 12) and/or remote (figures 3 - 8) equipment will be provided. On the former or or both of them the transducers and the commutators, as well as, possibly the demi-remote equipment, functioning as interface, even in regard of signals transmission networks, could be provided. Every connoisseur, even if not expert of the art, may easily imagine all the contemporary and alternative uses which can be made of the equipments according to the invention.

## Claims

1. Device for surveying, particularly permanently, in real time, signals having diagnostic meaning and for revealing them at a close and/or remote distance, in any perceptible and/or technical available form, comprising a carrier (10) substantially in the form of wrist watch, at least a sensory equipment (100) providing signals having diagnostic meaning, at least a directly or indirectly emitter (500) displaying a signal in any percepible form, and a power economiser (18), said sensor (1) providing signals having diagnostic meaning, emerging from the inner side of the bracelet, band worn on the arm, and particularly from its casing (21) characterized in that said sensor is applied on the end of a flexible arm (9), in order that, by wearing the bracelet, the sensor (1) adheres to the subject's skin, with a pressure suitable to provide a correct detection.

2. Device, as claimed in claim 1, characterized in that the carrier or holder (10), substantially in the form of wrist watch, has the sensory equipment (1), providing signals having diagnostic meaning, which emerges from the inner side of the bracelet (band worn on the arm) and particularly from its casing (21).

3. Device, as claimed in c!aim 2, characterized in that said sensor (1) applied to the end of a flexible arm (9) is a sensor of the body temperature.

4. Device, as claimed in claim 3, characterized in that the body temperature sensor (1) is an electronic thermoprobe.

5. Device, as claimed in claim 4, characterized in that said electronic temperature probe (1) is of the demi-cylindrical kind and that it is placed on the flexible arm (9) in such a way that the skin contact area (1') of the user corresponds to the even surface, wherein the sensing cells of the same probe are placed.

6. Device, as claimed in claim 4, characterized in that the thermoprobe (1) is provided with a flexible bell-like enclosure (11') in the form of a corolla of petals (111'), whereby such petals may adhere to the skin, due to their own elasticity and to the pressure applied, substantially providing the thermoprobe (1) with a constant temperature environment.

7. Device, as claimed in claims from 1 to 6, characterized in that such flexible arm (9) cooperates with a battery (19) disconnecting switch, controlled by the swinging of the flexible arm (9), whereby the switch is disconnected when the flexible arm (9) is released.

8. Device, as claimed in claim 1, characterized in that more sensory equipments (1, 101, 201, 301, 401), providing signals having diagnostic meaning, include each a pole (1, 101, 201, 301, 401) of a commutation switch (601).

9. Device, as claimed in claim 8, characterized in that at least one pole (501) of the commutation switch.(601) is comprised by a plug, for the connection of at least one pole (701) of a sensory equipment (701') provided at the free pole of a cable (702).

10. Device, as claimed in claim 1, characterized in that the disclosing equipment (5, 105, 205, 305, 405) displaying a signal in any percepible form comprises at least a transducer (5', 105', 205', 305', 405') of the detected signal and at least one emitter (5'', 105'', 205'', 305'', 405'') of the converted signal.

11. Device, as claimed in claim 10, characterized in that the disclosing equipment (5, 105, 205, 305, 405) displaying a signal in any percepible form comprises more transducers (5', 105', 205', 305', 405') of the disclosed signal and a commutation switch (605), having a number of poles (5''', 105''', 205''', 305''', 405'''), equal to the number of transducers, provided upstream of said transducers.

12. Device, as claimed in claim 11, characterized in that, at least one of the transducers (505'), is a radio-frequency transducer provided downstream with an antenna (0).

13. Device, as claimed in claim 12, characterized in that at least a radio-frequency transducer (505') is a transreceiver transducer.

14. Device, as claimed in claim 10, characterized in that the transducer (5', 105', 205', 305', 405') of the disclosed signal is placed on at least a demi-remote equipment (55) and/or at least an equipment (555) remove from the bracelet component (10).

15. Device, as claimed in claim 14, characterized in that more transducers (5', 105', 205', 305', 405') of the disclosed signal and a commutation switch (605) having a number of poles (5''', 105''', 205''', 305''', 405''') equal to the number of transducers, provided upstream of transducers, are provided on the demi-remote equipment (55) and/or on the remote equipment (555).

16. Device, as claimed in claim 15, characterized in that at least one of transducers (505') is a radio-frequency transducer provided downstream with an antenna (0, 0')).

17. Device, as claimed in claim 16, characterized in that at least a a radio-frequency transducer (505') is a transreceiver transducer.

18. Device, as claimed in one or more of claims from 12 to 16, characterized in that the demi-remote equipment (55) is is a power repeater equipment, whereby to limit the power consume in the disclosing equipment (10).

19. Device, as claimed in claim 18, characterized in that the demi-remote equipment (55) is provided with means connectable to any network such as power, telephone, radio frequency, digital, optical-fibre network and the like.

## Patentansprüche

1. Einrichtung zur Erfassung, insbesondere der Dauerechtzeiterfassung von Signalen mit diagnostischer Relevanz, zur Nah- und/oder Fernanzeige, in all ihren erfaßbaren und/oder technisch verfügbaren Formen, die im wesentlichen einen armbanduhrartigen Halter (10) mit mindestens einem Sensorenabschnitt (100) zur Erfassung von Signalen mit diagnostischer Relevanz, mindestens einen direkten oder indirekten Sender (500) zur perzeptiven Signalanzeige in irgend einer Form, und den Stromsparer (18) umfasst, wobei der genannte Sensor (1), die auf der Armbandinnenseite, insbesondere im Gehäuse (21) entstehende Signale mit diagnostischer Relevanz erfaßt, dadurch gekennzeichnet, daß der genannte Sensor am Ende eines elastischen Armes (9) eingerichtet ist sodaß der Sensor (1) beim Tragen des Armbandes, die Haut des Trägers mit einem eine angemessenen Signalerfassung gewährleistenden Druck, berührt.

2. Einrichtung nach Anspruch 1, dadurch gekennzeichnet, daß der im wesentlichen armbanduhrförmige Halter (10) einen Signale mit diagnostischer Relevanz erzeugenden Sensorenabschnitt (1) besitzt, welcher aus der Armbandinnenseite, insbesondere aus dem Gehäuse (21), des um den Arm geschlossenen Armbandes hervorragt.

3. Einrichtung nach Anspruch 2, dadurch gekennzeichnet, daß der am Ende des elastischen Arms (9) angeordnete genannte Sensor (1) ein Körpertemperatursensor ist.

4. Einrichtung nach Anspruch 3, dadurch gekennzeichnet, daß der Körpertemperatursensor eine elektronische Temperatursonde ist.

5. Einrichtung nach Anspruch 4, dadurch gekennzeichnet, daß die genannte elektronische Temperatursonde (1) der halbzylindrischen Art ist und auf dem elastischen Arm (9) angeordnet ist, sodaß die Benützerhaut-Berührungsfläche (1') mit der Ebene übereinstimmt auf der die Meßfühler der genannten Sonde angeordnet sind.

6. Einrichtung nach Anspruch 4, dadurch gekennzeichnet, daß die elektronische Temperatursonde (1) mit einer elastischen, blumenkronenartigen Glocke (11') ausgerüstet ist, wobei die Blumenblätter (111') durch die eigene Elastizität oder den angewandten Druck die Haut berühren, womit dies Temperatursonde (1) im wesentlichen in einer gleichmäßige Temperaturumgebung zu liegen kommt.

7. Einrichtung nach den Ansprüchen von 1 bis 6, dadurch gekennzeichnet, daß der genannte elastische Arm (9), mit einem durch die Ausschwenkung des elastischen Armes (9) betätigten Batterie(19)ausschalter (18) zusammenwirkt, wobei der Entspannungslage des elastischen Armes (9) Schalterunterbruch entspricht.

8. Einrichtung nach Anspruch 1, dadurch gekennzeichnet, daß jeder von mehreren Signale mit diagnostischer Relevanz erzeugenden Sensorenteilen (1, 101, 201, 301, 401) einen Schalter(601)pol (1, 101, 201, 301, 401) aufweist.

9. Einrichtung nach Anspruch 8, dadurch gekennzeichnet, daß mindestens ein Pol (501) des Schalters (601) von einem Stecker zur Verbindung mindestens eines Pols (701) eines an den freien Pol des Kabels (702) angeordneten Sensorabschnittes (701'), umfaßt ausgebildet ist.

10. Einrichtung nach Anspruch 1, dadurch gekennzeichnet, daß die Signale von irgenwelcher wahrnehmbaren Form erfassende Anzeigeausrüstung (5, 105, 205, 305, 405) mindestens einen Messwertwandler (5', 105', 205', 305', 405') für das empfangene Signal und mindestens einen Sender (5'', 105'', 205'', 305'', 405'') für das konvertierte Signal, umfaßt.

11. Einrichtung nach Anspruch 1, dadurch gekennzeichnet, daß die erfaßte Signale verschiedenartiger Form aufzeigende Anzeigeausrüstung (5, 105, 205, 305, 405), zumindest mehrere Messwertwandler (5', 105', 205', 305', 405') des aufgezeigten Signals und einen Schalter (605) mit einer Pol(5''', 105''', 205''', 305''', 405''')anzahl, gleich der und oberhalb der genannten Messwertwandlern angeordneten Messerwertwandleranzahl umfaßt.

12. Einrichtung nach Anspruch 11, dadurch gekennzeichnet, daß mindestens einer der Messwertwandler (505') ein Hochfrequenz-Messwertwandler mit einer unterhalb angeordneten Antenne (0) ist.

13. Einrichtung nach Anspruch 12, dadurch gekennzeichnet, daß mindestens ein Hochfrequenz-Messwertwandler ein Sende-Empfänger-Messwertwandler ist.

14. Einrichtung nach Anspruch 10, dadurch gekennzeichnet, daß der Messwertwandler (5', 105', 205', 305', 405') des erfaßten Signals mindestens auf einer halbentfernten Einheit (55) und/oder mindestens auf einer vom armbanduhrförmigen Halter (10) entfernten Einheit (555) angeordnet ist.

15. Einrichtung nach Anspruch 14, dadurch gekennzeichnet, daß mehrere Messwertwandler (5', 105', 205', 305', 405') des erfaßten Signals und ein oberhalb angeordneter Schalter (605) mit gleicher Pol(5''', 105''', 205''', 305''', 405''' )anzahl wie die Messwertwandleranzahl auf der halbentfernten Einheit (55) und/oder auf der entfernten Einheit (555) eingerichtet sind.

16. Einrichtung nach Anspruch 15, dadurch gekennzeichnet, daß mindestens einer der Messwertwandler (505') ein Hochfrequenz-Messwertwandler ist, der unterhalb eine Antenne (0, 0') aufweist.

17. Einrichtung nach Anspruch 16, dadurch gekennzeichnet, daß mindestens einer der Hochfrequenz-Messwertwandler (505') auch ein Sender-Empfänger Messwertwandler ist.

18. Einrichtung nach den Ansprüchen von 12 bis 16, dadurch gekennzeichnet, daß die halbentfernte Einheit (55) ein Leistungsverstärker ist, der geeignet ist, den Energieverbrauch auf der Anzeigeeinheit (10) zu begrenzen.

19. Einrichtung nach Anspruch 16, dadurch gekennzeichnet, daß die halbentfernte Einheit (55) mit Mitteln ausgerüstet ist, die mit irgend einem Netzwerk wie Kraft-, Telephon-, Hochfrequenz-, Digitalen und Optische Netzwerk und Ähnlichem verbindbar sind.

## Revendications

1. Dispositif pour relever, notamment en permanence, en temps réel, des signaux ayant une importance diagnostique et pour les révéler à une distance éloignée et/ou proche, dans toutes les formes perceptibles et/ou techniques disponibles, comprenant un support (10) essentiellement en forme de montre-bracelet, au moins un organe (100) senseur relevant les signaux ayant une importance diagnostique, au moins un émetteur (500) révélant directement ou indirectement un signal dans toutes les formes perceptibles, et un économiseur (18) de puissance, ledit senseur (1) relevant les signaux ayant une importance diagnostique, sortant de la face intérieure du bracelet, qui est une bande portée au poignet, et notamment de sa boîte (21), caractérisé en ce que ledit senseur est appliqué à l'extrémité d'un bras (9) flexible, de sorte que le senseur (1) adhère à la peau du sujet portant le bracelet avec une pression apte à donner un relevé correct.

2. Dispositif, selon la revendication 1, caractérisé en ce que le support (10) ou soutien, essentiellement en forme de montre-bracelet, présente l'organe (1) senseur relevant les signaux ayant une importance diagnostique, sortant de la face intérieure du bracelet (bande portée au poignet) et notamment de sa boîte (21).

3. Dispositif, selon la revendication 2, caractérisé en ce que ledit senseur (1), appliqué à l'extrémité d'un bras (9) flexible, est un senseur de la température corporelle.

4. Dispositif, selon la revendication 3, caractérisé en ce que le senseur (1) de la température corporelle est une sonde thermique électronique.

5. Dispositif, selon la revendication 4, caractérisé en ce que ledit senseur (1) est du type semi-cilyndrique et en ce qu'il est monté sur le bras (9) flexible, de sorte que la surface (1') de contact avec la peau de l'utilisateur correspond à la surface plane où on prévoit les cellules captrices du senseur-lui-même.

6. Dispositif, selon la revendication 4, caractérisé en ce que le senseur (1) de la température est purvu d'une enceinte flexible à cloche (11'), en forme de corolle, où les pétales (111') adhèrent à la peau grâce à l'élasticité propre et à la pression appliquée, le senseur (1) de température étant essentiellement pourvu d'un environnement à température constante.

7. Dispositif, selon les revendications de 1 à 6, caractérisé en ce que ledit bras (9) flexible coopère avec un interrupteur (18) de mise hors circuit de la batterie (19), commandé par l'excursion du bras (9) flexible, où, à bras (9) flexible libre, l'interrupteur est débranché.

8. Dispositif, selon la revendication 1, caractérisé en ce que chacun des plusieurs organes senseurs (1, 101, 201, 301, 401) relevant les signaux ayant une importance diagnostique, inclut un pôle (1, 101, 201, 301, 401) d'un commutateur (601).

9. Dispositif, selon la revendication 8, caractérisé en ce que au moins un pôle (501) du commutateur (601) est constitué par une prise, pour la connexion d'au moins un pôle (701) d'un organe senseur (701') appliqué au pôle libre d'un câble (702).

10. Dispositif, selon la revendication 1, caractérisé en ce que l'organe (5, 105, 205, 305, 405) révélant un signal perceptible dans toutes les formes, comprend plusieurs transducteurs (5', 105', 205', 305', 405') du signal relevé et au moins un émetteur (5'', 105'', 205'', 305'', 405'') du signal transduit.

11. Dispositif, selon la revendication 10, caractérisé en ce que l'organe (5, 105, 205, 305, 405) révélant un signal dans toutes les formes perceptibles, comprend plusieurs transducteurs (5', 105', 205', 305', 405') du signal relevé et un commutateur, (605) ayant un nombre de pôles (5''', 105''', 205''', 305''', 405''') égal au nombre des transducteurs, placé en dessus desdits transducteurs.

12. Dispositif, selon la revendication 11, caractérisé en ce que, au moins un des transducteurs (505') est un transducteur à radiofréquences pourvu, en dessous, d'une antenne (0).

13. Dispositif, selon la revendication 12, caractérisé en ce que au moins un transducteur (505') à radiofréquences est émetteur- récepteur.

14. Dispositif, selon la revendication 10, caractérisé en ce que le transducteur (5', 105', 205', 305', 405') du signal révélé est disposé sur au moins un système (55) demi-éloigné et/ou au moins sur un système (555) éloigné de l'élément (10) à bracelet.

15. Dispositif, selon la revendication 14, caractérisé en ce que, sur le système (55) demi-éloigné et sur le système (555) éloigné, on prévoit plusieurs transducteurs (5', 105', 205', 305', 405') du signal révélé et un commutateur (605) ayant un nombre de pôles (5''', 105''', 205''', 305''', 405''') égal au nombre des transducteurs, disposé en dessus des transducteurs.

16. Dispositif, selon la revendication 15, caractérisé en ce que au moins un des transducteurs (505') est un transducteur à radiofréquences pourvu, en dessous, d'une antenne (0,0').

17. Dispositif, selon la revendication 16, caractérisé en ce que au moins un transducteur (505') à radiofréquences est un transducteur émetteur-récepteur.

18. Dispositif, selon une ou plusieures revendications de 12 à 16, caractérisé en ce que le système (55) demi-éloigné est un système répéteur de puissance, prévu pour la limitation de la consommation d'énergie sur l'unité (10) révélatrice.

19. Dispositif, selon la revendication 18, caractérisé en ce que le système (55) demi-éloigné est pourvu de moyens connectables à tout réseau de distribution électrique, téléphonique, de radiofréquences, digital, fibre optiques et similaires.
